# EUROPEAN PATENT APPLICATION

(11) **EP 4 269 606 A1**
(43) Date of publication of application: **01.11.2023**
(21) Application number: 21911509.4
(22) Date of filing: 22.12.2021
(51) Int. Cl.: C12Q 1/68, G01N 33/569, A61K 35/28, A61P 37/06

(54) **MARKER FOR SELECTING HIGH-QUALITY STEM CELLS, AND METHOD FOR SELECTING HIGH-QUALITY STEM CELLS USING SAME**

(30) Priority: 22.12.2020 KR 20200180455
(71) Applicant: Cell2in, Inc., Seoul 03080 (KR)
(72) Inventor: KANG, Heunsoo, Seoul 06272 (KR); SHIN, Ji Woong, Seoul 03438 (KR); KANG, Hyewon, Seoul 08303 (KR); KIM, Hye Mi, Seoul 01054 (KR); LEE, Kibaek, Seoul 07229 (KR); JEON, Hatnim, Seoul 05227 (KR)
(74) Representative: Müller, Christian Stefan Gerd
(86) International application number: PCT/KR2021/019582
(87) International publication number: WO 2022/139442

(57) **Abstract**

The present invention relates to a composition and a kit for assessing the quality of stem cells, and a method for producing stem cells, and may provide a cell therapy product and a composition for preventing or treating immune-related disease.

## Description

### Technical Field

The present invention relates to a cell surface marker protein for selection of high-quality stem cells and a method of selecting high-quality stem cells using the marker protein.

### Background Art

Stem cells are undifferentiated cells that have the ability to self-renew and differentiate into various types of human cells. When stem cells are applied as therapeutic agents, they are thought to provide anti-inflammatory effects and mediate repair through five major mechanisms: restoration of damaged tissue to a normal state, recruitment of cells such as endothelial progenitor cells required for tissue growth, tissue remodeling by scar formation, inhibition of apoptosis, and differentiation into tissues.

In addition, stem cells are known to have low immunogenicity due to a relatively small number of MHC molecules expressed on their surfaces. It has also been found that stem cells secrete chemokines that evade immune responses and promote tolerance of new tissue. This allows for allogeneic treatments to be performed without a high immune rejection risk. The potency of some stem cells can be affected by the method of delivery.

Stem cells include embryonic stem cells, which can be made from human embryos, and adult stem cells, such as bone marrow cells that constantly produce blood cells. Embryonic stem cells can differentiate into all cells and tissues constituting the human body, but their use is limited for ethical reasons, whereas adult stem cells are extracted from umbilical cord blood or adult bone marrow and blood, and divided into mesenchymal stem cells and hematopoietic stem cells.

Thereamong, mesenchymal stem cells (MSCs) are stem cells present throughout the body, including bone marrow, which are capable of differentiating into various cell lineages such as adipocytes, osteocytes, and chondrocytes. Since it was discovered by Alexander A. Maximow (1874-1928) that there are progenitor cells capable of differentiating into blood cells in the mesenchyme, mesenchymal stem cells were first isolated from the stroma of bone marrow by Alexander J. Friedenstein (1924-1998) in 1976 and were named precursors for fibroblasts. In the 1990s, many researchers found that mesenchymal stem cells had the ability to differentiate into chondrocytes, myocytes, adipocytes, osteoblasts, and the like, and the term "mesenchymal stem cells" began to be used. Since then, mesenchymal stem cells have been isolated from a number of species, including humans, mice, rats, dogs, goats, rabbits, and cats.

In cell therapy technology using mesenchymal stem cells, it is important to use only high-purity and high-quality cells. This therapy technology has been attempted to treat intractable diseases such as various inflammatory diseases. Therefore, there is a need for markers for assessing the performance of mesenchymal stem cells for application to mesenchymal stem cell therapy products.

### DISCLOSURE

### Technical Problem

An object of the present invention is to provide a composition and a kit for assessing the quality of stem cells.

Another object of the present invention is to provide a method for selecting activated stem cells that may be used as a cell therapy product.

Still another object of the present invention is to provide a cell therapy product and a composition for preventing or treating immune-related disease, which contain stem cells selected by the method of the present invention.

However, objects to be achieved by the present invention are not limited to the above-mentioned objects, and other objects not mentioned herein will be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments. Unless otherwise stated in the specification, all the scientific and technical terms used in the specification have the same meanings as commonly understood by those skilled in the technical field to which the present invention pertains.

According to one embodiment of the present invention, there is provided a composition for assessing the quality of stem cells, the composition containing an agent for measuring the expression level of a CD71 (Cluster of Differentiation 71) protein or a gene encoding the same.

In the present invention, the "CD71 protein" is also referred to as a transferrin receptor (TFRC), and the CD71 protein interacts with a transferrin protein, which binds to iron flowing through the blood cell flow, and serves to supply iron to cells through a process called receptor-mediated transferrin endocytosis. The CD71 protein may be represented by SEQ ID NO: 1. The gene encoding the CD71 protein is located on chromosome 3 in humans and chromosome 16 in mice.

In the present invention, the agent for measuring the expression level of the CD71 protein may comprise at least one selected from the group consisting of antibodies, oligopeptides, ligands, peptide nucleic acids (PNAs), and aptamers, which bind specifically to the protein, without being limited thereto.

In the present invention, the "antibody" refers to a substance that specifically binds to an antigen and causes an antigen-antibody reaction. For the purposes of the present invention, the antibody refers to an antibody that specifically binds to the CD71 protein.

Examples of the antibody of the present invention include all of polyclonal antibodies, monoclonal antibodies, and recombinant antibodies. This antibody may be readily produced using techniques well known in the art. For example, a polyclonal antibody may be produced by a method well known in the art, which includes a process of obtaining a serum containing the antibody by injecting the antigen of the protein into an animal and collecting blood from the animal. This polyclonal antibody may be produced from any animal such as goat, rabbit, sheep, monkey, horse, pig, cow, dog, or the like. In addition, a monoclonal antibody may be produced using the hybridoma method well known in the art (see Kohler and Milstein (1976) European Journal of Immunology 6:511-519), or the phage antibody library technology (see Clackson et al, Nature, 352:624-628, 1991; Marks et al, J. Mol. Biol., 222:58, 1-597, 1991). The antibody produced by the above method may be isolated and purified using a method such as gel electrophoresis, dialysis, salt precipitation, ion exchange chromatography, or affinity chromatography. In addition, examples of the antibody of the present invention include not only a complete form having two full-length light chains and two full-length heavy chains, but also functional fragments of an antibody molecule. "Functional fragment of an antibody molecule" refers to a fragment having at least an antigen-binding function, and examples thereof include Fab, F(ab'), F(ab')2 and Fv.

In the present invention, "PNA (Peptide Nucleic Acid)" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, PNA is used for molecular biology, diagnostic assays and antisense therapies. The PNA is disclosed in detail in the literature [Nielsen PE, Egholm M, Berg RH, Buchardt O (December 1991). "Sequence-selective recognition of DNA by strand displacement with a thymine-substituted polyamide". Science 254(5037): 1497-1500].

In the present invention, the "aptamer" refers to an oligonucleotide or a peptide molecule, and the general contents of the aptamer are disclosed in detail in the literature [Bock L C et al., Nature 355(6360):5646(1992); Hoppe-Seyler F, Butz K "Peptide aptamers: powerful new tools for molecular medicine". J Mol Med. 78(8):42630(2000); Cohen B A, Colas P, Brent R. "An artificial cell-cycle inhibitor isolated from a combinatorial library". Proc Natl Acad Sci USA. 95(24): 142727(1998)].

In the present invention, the agent for measuring the expression level of the gene encoding CD71 may comprise at least one selected from the group consisting of primers, probes, and antisense nucleotides, which bind specifically to the gene.

In the present invention, the term "primer" refers to a fragment that recognizes a target gene sequence, and comprises a pair of forward and reverse primers, but is preferably a pair of primers providing analysis results with specificity and sensitivity. When the nucleic acid sequence of the primer is a sequence inconsistent with the non-target sequence present in the sample, and thus the primer amplifies only the target gene sequence containing the complementary primer binding site without inducing non-specific amplification, high specificity may be imparted.

In the present invention, the term "probe" refers to a substance capable of binding specifically to a target substance to be detected in a sample, and refers to a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, an RNA or a DNA, and most preferably PNA. More specifically, the probe is a biomolecule derived from an organism or an analogue thereof, or is produced *in vitro.* Examples of the probe include an enzyme, a protein, an antibody, a microorganism, an animal and/or plant cell and organ, a neuron, DNA, and RNA. Examples of the DNA include cDNA, genomic DNA, and an oligonucleotide, examples of the RNA include genomic RNA, mRNA and an oligonucleotide, and examples of the protein include antibodies, antigens, enzymes, peptides, and the like.

In the present invention, the term "LNA (locked nucleic acid)" refers to a nucleic acid analogue containing a 2'-O or 4'-C methylene bridge [J Weiler, J Hunziker and J Hall Gene Therapy (2006) 13, 496.502]. LNA nucleosides comprise the common nucleobases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand. In the present invention, the term "antisense" means an oligomer that has a nucleotide sequence and a backbone between subunits, wherein an antisense oligomer is hybridized with the target sequence in the RNA by Watson-Crick base pairing to typically allow the formation of the mRNA and RNA:oligomer heterodimers in the target sequence. The oligomer may have an accurate or approximate sequence complementarity to the target sequence.

Information on the sequence of the CD71 protein according to the present invention is known. Thus, based on this information, any person skilled in the art will be able to easily design a primer, probe or antisense nucleotide that binds specifically to the gene encoding the CD71 protein.

In the present invention, when the expression level of the CD71 protein or the gene encoding the same in the stem cells is lower than a control, the stem cells may be determined to be of high quality. Here, the control may be the expression level of the CD71 protein or the gene encoding the same in any stem cells, or may be the average value of the expression level of the CD71 protein or the gene encoding the same in the stem cells, without being limited thereto. In the present invention, the "stem cell" refers to a cell having the ability to differentiate into two or more types of new cells while having self-renewal ability. Stem cells may be classified, according to the differentiation potential, into totipotent stem cells, pluripotent stem cells, multipotent stem cells, and unipotent stem cells, and may be classified, according to the tissue from which they originate, into adult stem cells, embryonic stem cells, and dedifferentiated stem cells. The totipotent or pluripotent stem cells refer to cells having the ability to differentiate into all tissues or cells constituting a living body. In addition, the multipotent stem cells refer to cells having the ability to differentiate into not all types but multiple types of tissues or cells. The unipotent stem cells refer to cells having the ability to differentiate into a specific type of tissue or cell. Examples of the totipotent stem cells include embryonic stem cells (ES cells), undifferentiated embryonic germ cells (EG cells), and induced pluripotent stem cells (iPS cells). Examples of the multipotent stem cells include adult stem cells such as mesenchymal stem cells (derived from adipose, bone marrow, cord blood or umbilical cord), hematopoietic stem cells (derived from bone marrow or peripheral blood), nervous system stem cells, and reproductive stem cells. In addition, examples of the unipotent stem cells include committed stem cells which are usually present with a low division capacity, but once activated, vigorously divide and self-renew without differentiation potential. Adult stem cells are cells that exist in small amounts in each tissue of the body, and are cells constituting a specific tissue. In addition, the adult stem cells provide cells necessary for the body to always be maintained in a healthy state, and when tissue damage occurs, repair the damaged tissue by differentiation together with stem cells that migrated from other tissues to the damaged area. Those skilled in the art to which the present invention pertains generally recognize that stem cells having a smaller size and fewer intracellular granules (lower cell granularity) are "higher-quality stem cells" (Lengefeld J et al., Cell size is a determinant of stem cell potential during aging. Sci Adv. 2021 Nov 12;7(46): Epub 2021 Nov 12.). Also, stem cells having better colony forming ability (the number of colony forming units) are regarded to be "higher-quality stem cells" (Christine Fehrer et al., Mesenchymal stem cell aging. Experimental Gerontology. Volume 40, Issue 12, December 2005, Pages 926-930).

The term "high quality" means that the stem cells are highly efficient and highly effective as a cell therapy product. Therefore, the criterion for selecting the high-quality stem cells may be 1) cell size and granularity, 2) the colony forming ability, or 3) cell protein expression markers. The above expression markers may be proteins (positive markers) whose expression increases by 30% or more in a high-quality stem cell population compared to in a general stem cell population, or proteins (negative markers) whose expression decreases by 30% or more in a high-quality stem cell population compared to in a general stem cell population. For example, it is known that the expression of vascular endothelial growth factor (VEGF) increases in young and healthy cells. In this case, the VEGF may be defined as a positive marker of high-quality stem cells. Alternatively, it is known that the expression of IL-6 or IL-8 increases as cells become senescent (Jing Liu et al., Senescence in Mesenchymal Stem Cells: Functional Alterations, Molecular Mechanisms, and Rejuvenation Strategies. Front. Cell Dev. Biol., 05 May 2020). In this case, cells with lower expression of IL-6 or IL-8 can be said to be young and healthy, and IL-6 or IL-8 may be defined as a negative marker of high-quality stem cells.

In the present invention, the stem cells may be mesenchymal stem cells (MSCs), without being limited thereto.

In the present invention, the term "mesenchymal stem cells" refers to cells having the ability (plasticity) to differentiate into various mesenchymal tissues while maintaining sternness and self-renewal. Thus, the mesenchymal stem cells selected by measuring their quality using the composition of the present invention are highly efficient and highly effective as a cell therapy product.

In the present invention, the mesenchymal stem cells may be any one or more cell types selected from the group consisting of human embryonic stem cell-derived mesenchymal stroma cells (hES-MSCs), umbilical cord mesenchymal stem cells (UC-MSCs), umbilical cord blood mesenchymal stem cells (UCB-MSCs), bone marrow mesenchymal stem cells (BM-MSCs), adipose-derived mesenchymal stem cells (ADMSCs), muscle-derived mesenchymal stem cells, nerve-derived mesenchymal stem cells, skin-derived mesenchymal stem cells, amnion-derived mesenchymal stem cells, and placenta-derived mesenchymal stem cells, without being limited thereto.

In the present invention, the mesenchymal stem cells may be derived from a biological sample, preferably a human biological sample, such as bone marrow, trabecular bone, periosteum, pulp, tendon, adipose tissue, blood, umbilical cord, placenta, fetal yolk sac, or skin (dermis), without being limited thereto.

As used herein, the term "biological sample" or "sample" refers to any material, tissue or cell obtained from a biological source, for example, an organism such as an animal or human subject, a cell culture, a tissue sample, or the like. A biological sample from an animal or human subject may include one or more tissue types and cells of one or more tissue types. A biological sample from an animal or human subject may include any stem cells isolated according to any method known in the art, and preferably, the stem cells may be mesenchymal stem cells.

In the present invention, the mesenchymal stem cells are capable of generating cells of mesenchymal lineages, typically of two or more mesenchymal lineages, more typically three or more mesenchymal lineages, e.g., osteochondroblastic (bone and cartilage), osteoblastic (bone), chondroblastic (cartilage), myocytic (muscle), tenocytic (tendon), fibroblastic (connective tissue), adipocytic (fat) and stromogenic (marrow stroma) lineage.

In the present invention, "stem cell quality" includes without limitation various states of stem cells in a stem cell sample, but preferably, relates to whether the stem cell sample is applicable clinically (e.g., cell therapy product).

In the present invention, the stem cell quality may mean the degree of differentiation (e.g., undifferentiated or osteocyte differentiation) of the stem cells, or the expression levels of growth factors, cytokines, and other proteins, but is not limited thereto as long as it relates to requirements suitable for substantially using the stem cells as a cell therapy product.

In the present invention, "assessment" or "determination" includes quantitative and/or qualitative analysis, which includes the detection of presence or absence, or the measurement of expression level and concentration. Such methods are known in the art, and those skilled in the art will be able to select an appropriate method for practicing the present invention.

Another embodiment of the present invention is directed to a kit for assessing the quality of stem cells, the kit comprising the composition for assessing the quality of stem cells according to the present invention. The quality of stem cells may be measured using the kit for assessing the quality of stem cells.

In the present invention, details regarding the stem cells, the stem cell quality and the measurement overlap with those described above, and thus detailed description thereof will be omitted to avoid excessive complexity of the specification.

In the present invention, the kit may be an RT-PCR kit, a DNA chip kit, an ELISA kit, a protein chip kit, or a rapid kit, without being limited thereto.

The kit for assessing the quality of stem cells according to the present invention may further comprise one or more other component compositions, solutions or devices suitable for the analysis method.

For example, the kit for assessing the quality of stem cells according to the present invention may further comprise essential elements required for performing a reverse transcription polymerase chain reaction. The reverse transcription polymerase chain reaction kit comprises a primer pair specific for the gene encoding the marker protein. The primer is an oligonucleotide having a sequence specific for the nucleic acid sequence of the gene, and may have a length of about 7 bp to 50 bp, more preferably about 10 bp to 30 bp. The kit may also comprise a primer specific for the nucleic acid sequence of the control gene. In addition, the reverse transcription polymerase chain reaction kit may comprise test tubes or other appropriate containers, reaction buffers (at various pHs and magnesium concentrations), deoxynucleotides (dNTPs), enzymes such as Taq-polymerase and reverse transcriptase, DNase and/or RNase inhibitors, DEPC water, sterile water, and the like.

In addition, the kit for assessing the quality of stem cells according to the present invention may comprise essential elements required for performing DNA chip assay. The DNA chip kit may comprise a substrate to which a cDNA or oligonucleotide corresponding to a gene or a fragment thereof is attached, and a reagent, an agent, an enzyme, and the like for producing a fluorescent-labeled probe. The substrate may also comprise a cDNA or oligonucleotide corresponding to a control gene or a fragment thereof.

In addition, the kit for assessing the quality of stem cells according to the present invention may comprise essential elements required for performing ELISA. The ELISA kit comprises an antibody specific for the protein. The antibody has high specificity and affinity for the marker protein and little cross-reactivity with other proteins, and is a monoclonal antibody, a polyclonal antibody or a recombinant antibody. The ELISA kit may also comprise an antibody specific for the control protein. In addition, the ELISA kit may comprise reagents capable of detecting the bound antibody, such as a labeled secondary antibody, chromophores, an enzyme (e.g., conjugated to an antibody) and substrates thereof, or other substances that may bind to the antibody.

In the kit for assessing the quality of stem cells according to the present invention, a support for the antigen-antibody binding reaction may be selected from among nitrocellulose membranes, PVDF membranes, well plates made of polyvinyl or polystyrene resin, and slide glasses, without being limited thereto.

In addition, in the kit for assessing the quality of stem cells according to the present invention, the secondary antibody is preferably labeled with a conventional chromogenic agent that participates in a chromogenic reaction. The chromogenic agent may be selected from among fluoresceins and dyes, without being limited thereto. The fluoresceins may be, for example, horseradish peroxidase (HRP), alkaline phosphatase, colloid gold, poly-L-lysine-fluorescein isothiocyanate (FITC), and rhodamine-B-isothiocyanate (RITC).

In addition, in the kit for assessing the quality of stem cells according to the present invention, a chromogenic substrate for inducing color development is preferably used depending on the label that participates in the chromogenic reaction. The substrate may be selected from among 3,3',5,5'-tetramethylbenzidine (TMB), 2,2'-azino-bis(3-ethylbenzothiazoline)-6-sulfonic acid (ABTS), o-phenylenediamine (OPD), and the like. In this case, the chromogenic substrate is more preferably provided in a state in which it is dissolved in a buffer solution (0.1 M NaAc, pH 5.5). The chromogenic substrate such as TMB is degraded by HRP used as a label for the secondary antibody conjugate to generate a chromogenic deposit, and the deposition level of the chromogenic deposit is checked visually, thereby detecting the presence or absence of the marker protein.

In the kit for assessing the quality of stem cells according to the present invention, the washing solution preferably includes a phosphate buffer solution, NaCl, and Tween 20, and is more preferably a buffer solution (PBST) composed of a 0.02 M phosphate buffer solution, 0.13 M NaCl, and 0.05% Tween 20. After the secondary antibody is allowed to react with the antigen-antibody conjugate after the antigen-antibody binding reaction, a proper amount of the washing solution is added to the support to wash the substrate 3 to 6 times. A sulfuric acid solution (H₂SO₄) may be preferably used as the reaction stop solution.

Still another embodiment of the present invention is directed to a method for selection of activated stem cells, the method comprising steps of obtaining stem cells; and measuring the expression level of a CD71 (Cluster of Differentiation 71) protein or a gene encoding the same in the stem cells.

In the present invention, the step of obtaining stem cells may be performed by isolating the stem cells from a biological sample isolated from a subject.

In the present invention, the term "subject" refers to a subject having stem cells, includes a subject in need of stem cell treatment and a subject that simply donates stem cells, and may include both mammals and non-mammals. Here, examples of the mammals include, but are not limited to, humans, non-human primates such as chimpanzees, other ape or monkey species; livestock animals such as cattle, horses, sheep, goats, and pigs; domesticated animals such as rabbits, dogs or cats; laboratory animals, for example, rodents such as rats, mice, or guinea pigs. In addition, examples of the non-mammals in the present invention include, but are not limited to, birds or fish.

As used herein, the term "biological sample" or "sample" refers to any material, tissue or cell obtained from a biological source, for example, an organism such as an animal or human subject, a cell culture, a tissue sample, or the like. A biological sample from an animal or human subject may include one or more tissue types and cells of one or more tissue types. A biological sample from an animal or human subject may include any stem cells isolated according to any method known in the art, and preferably, the stem cells may be mesenchymal stem cells.

In the present invention, the biological sample may be derived from bone marrow, trabecular bone, periosteum, pulp, tendon, adipose tissue, blood, umbilical cord, placenta, fetal yolk sac, or skin (dermis), without being limited thereto.

In the present invention, the "isolation" is a process of isolating stem cells from a biological sample, and examples thereof include a method of isolating stem cells from adipose tissue using a manual method. This method is based on treatment of a lipoaspirate with 0.075% collagenase type I solution for 30 minutes at 37°C followed by centrifugation of the resulting suspension at 800g. After centrifugation, the cell suspension is divided into two fractions: the upper supernatant layer containing adipocytes, and the solution having blood cell contamination in the precipitate.

In the present invention, the agent for measuring the expression level of the CD71 protein may comprise at least one selected from the group consisting of antibodies, oligopeptides, ligands, peptide nucleic acids (PNAs), and aptamers, which bind specifically to the protein, without being limited thereto.

In the present invention, measurement of the expression level of the CD71 protein may be performed by at least one selected from the group consisting of protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), Western blotting, and enzyme-linked immunosorbent assay (ELISA), without being limited thereto.

In the present invention, the agent for measuring the expression level of the gene encoding the CD71 protein may be at least one selected from the group consisting of primers, probes, and antisense nucleotides, which bind specifically to the gene, without being limited thereto.

In the present invention, examples of analysis methods of measuring the amount of mRNA to confirm the presence or expression level of the gene encoding the CD71 protein include, but are not limited to, reverse transcription polymerase chain reaction (RT-PCR), competitive RT-PCR, real-time RT-PCR, RNase protection assay (RPA), Northern blotting, and DNA chip assay.

In the present invention, when the expression level of the CD71 protein or the gene encoding the same in the stem cells is lower than a control, the stem cells may be determined to be of high quality. Here, the control may be the expression level of the CD71 protein or the gene encoding the same in any stem cells, or may be the average value of the expression level of the CD71 protein or the gene encoding the same in the stem cells, without being limited thereto.

In the present invention, the phenotype of the selected stem cells may be CD71^{low} stem cells.

In the present invention, the phenotype of the stem cells may be classified into three groups based on the correlation with the expression level of the CD71 protein: a negative expression pattern of the cell surface molecule; a low expression level; and a high expression level. As an example, based on the expression level of the cell surface molecule, the top 30% may be defined as a high population, and the bottom 30% may be defined as a low population, without being limited thereto.

The selection method of the present invention may further comprise a step of measuring the oxidative stress of the stem cells, thereby increasing the accuracy of selection of activated stem cells suitable for use as a cell therapy product.

In the present invention, the "oxidative stress" refers to a situation in which cells are exposed to a state in which reactive oxygen species (ROS) are excessively accumulated due to an imbalance between generation and degradation of reactive oxygen species. Reactive oxygen species non-selectively attack proteins, lipids, and DNA, which are cell constituents, causing irreversible damage. Therefore, when the oxidative stress is additionally measured, it is possible to measure the quality of the stem cells.

In the present invention, the step of measuring the oxidative stress may be performed while measuring the intracellular glutathione (GSH) level.

In the present invention, glutathione is an antioxidant that plays an important role in redox reactions *in vivo.* When the glutathione level in cells is high, the cells may be defined as cells that are young, healthy, and highly therapeutically effective. Measurement of the intracellular glutathione level may be performed using a probe whose wavelength is different between when binding to glutathione and when not binding to glutathione. For example, the probe may be a small molecular probe (FreSHtracer) that can easily enter cells and organelles and can bind to the thiol (-SH) group of glutathione. When FreSHtrace binds to glutathione, it emits fluorescence at a wavelength of 510 nm (F510), and when it does not bind to glutathione, it emits fluorescence at a wavelength of 580 nm (F580). The amount of glutathione in cells may be measured using the fluorescence value of F510/F580 thus measured, and the F510/F580 is expressed as FR (FreSHtracer Ratio). The reaction between FreSHtracer and GSH is reversible and does not consume GSH in cells in measurement. In the present invention, the stem cells may be mesenchymal stem cells (MSCs).

In the present invention, the mesenchymal stem cells may be any one or more cell types selected from the group consisting of human embryonic stem cell-derived mesenchymal stroma cells (hES-MSCs), umbilical cord mesenchymal stem cells (UC-MSCs), umbilical cord blood mesenchymal stem cells (UCB-MSCs), bone marrow mesenchymal stem cells (BM-MSCs), adipose-derived mesenchymal stem cells (ADMSCs), muscle-derived mesenchymal stem cells, nerve-derived mesenchymal stem cells, skin-derived mesenchymal stem cells, amnion-derived mesenchymal stem cells, and placenta-derived mesenchymal stem cells, without being limited thereto.

In the present invention, the expression level of a growth factor in the stem cells selected as described above may be higher than the control, and the growth factor may be vascular endothelial growth factor (VEGF), without being limited thereto. Here, the control may be the expression level of VEGF in any stem cells, or the expression level of VEGF in stem cells showing a CD71^{low} phenotype, or the average value of the expression level of VEGF in stem cells, without being limited thereto.

In the present invention, the VEGF is a signaling protein that promotes the growth of new blood vessels, and forms a part of a mechanism that restores blood supply to cells and tissues for blood vessel formation and angiogenesis when oxygenated blood is deprived due to impaired blood circulation. Conventionally, it is known that VEGF expression increases in young and healthy stem cells.

In the present invention, the expression level of at least one of interleukin 6 (IL-6), interleukin 8 (IL-8) and heme oxygenase 1 (HMOX1) in the stem cells selected as described above may be lower than the control. Here, the control may be the expression level of IL-6, IL-8, or HMOX1 in any stem cells, or the expression level of IL-6, IL-8, or HMOX1 in stem cell showing a CD71^{high} phenotype, or the average value of the expression level of IL-6, IL-8, or HMOX1 in stem cells, without being limited thereto.

In the present invention, IL-6 is an anti-inflammatory cytokine that is produced in various cells such as T cells, B cells, macrophages, and fibroblasts. In addition to arresting the cell cycle and inducing differentiation from myeloid cell lines, IL-6 can play many important roles in key physiological systems including the nervous system. IL-8 is an essential factor involved in the activation of neutrophil migration during an acute inflammatory response. HMOX1 acts as an anti-inflammatory factor, like IL-6 and IL-8, and has an antioxidant function. Since HMOX1 is highly expressed in inflammatory cells, it has long been considered a target for treating chronic inflammatory and autoimmune diseases by immunologists (Nicole K. Campbell et al., Regulation of inflammation by the antioxidant haem oxygenase 1. Nature Reviews Immunology volume 21, pages 411-425. 2021).

In the present invention, details regarding the stem cells and the biological sample overlap with those described above, and thus detailed description thereof will be omitted to avoid excessive complexity of the specification.

Another embodiment of the present invention is directed to a cell therapy product containing, as an active ingredient, stem cells showing a CD71^{low} expression pattern, which are stem cells selected by the selection method of the present invention.

In the present invention, the "cell therapy product' refers to a pharmaceutical drug that is used for therapeutic, diagnostic and preventive purposes through a series of processes (e.g. the *in vitro* proliferation or selection of living autologous, allogenic or xenogenic cells, or the modification of biological properties of cells by other methods) intended to restore the function of cells and tissues. The United States (since 1993) and Korea (since 2002) have managed cell therapy products under the category of medical substances. The cell therapy product may be for tissue regeneration or organ function restoration.

The cell therapy product of the present invention may be administered through all general routes as long as it can reach the target tissue. It may be administered parenterally, for example, intraperitoneally, intravenously, intramuscularly, subcutaneously, or intradermally, without being limited thereto.

The cell therapy product of the present invention may be used for regeneration or protection of adipocytes, osteocytes, chondrocytes, muscle cells, nerve cells, cardiomyocytes, hepatocytes, beta cells, vascular cells, or lung cells. In addition, the cell therapy product of the present invention may be useful for any one selected from the group consisting of the treatment of lung diseases, the suppression or treatment of lung disease-induced inflammation, the regeneration of lung tissue, and the suppression of pulmonary fibrosis, and may be used to suppress or alleviate lung disease-induced inflammatory response and fibrosis. Further, the cell therapy product of the present invention may be used for the treatment of cardiovascular diseases or the regeneration of cartilage. In addition, the cell therapy product of the present invention can improve immunomodulative functions, or reduce one of immune response, immune cell penetration, or immunogenicity, and suppress inflammatory response.

The cell therapy product of the present invention may also be administered using any device capable of delivering the cell therapy product to target cells.

The cell therapy agent of the present invention may be contained in a therapeutically effective amount for treatment of a disease. The therapeutically effective amount refers to the amount of an active ingredient or pharmaceutical composition that induces a biological or medical response in a tissue system, animal or human, as determined by researchers, veterinarians, doctors or other clinicians, i.e., the amount that induces relief of the symptoms of the disease or disorder being treated.

It will be apparent to those skilled in the art that the cell therapy product contained in the composition of the present invention varies depending on the desired effect. Therefore, the optimal dose of the cell therapy product may be easily determined by those skilled in the art, and may be adjusted depending on various factors, including the type of disease, the severity of the disease, the contents of other ingredients in the composition, the type of formulation, the patient's age, weight, general state of health, gender and diet, time of administration, route of administration, rate of secretion of the composition, duration of treatment, and drugs used simultaneously therewith. It is important to administer the cell therapy product in the minimum amount that can exhibit the maximum effect without causing side effects, in view of all the above-described factors.

In the present invention, details regarding the stem cells overlap with those described above, and thus detailed description thereof will be omitted to avoid excessive complexity of the specification.

Another embodiment of the present invention is directed to a pharmaceutical composition for preventing or treating immune-related disease, the pharmaceutical composition containing, as an active ingredient, stem cells showing a CD71^{low} expression pattern, which are stem cells selected by the selection method of the present invention.

The stem cells of the present invention are characterized in that the expression level of VEGF or GSH therein is higher than the control, and the expression level of CD71, IL-6, IL-8, or HMOX1 therein is lower than the control. Due to these characteristics, the stem cells can regulate immunological tolerance and effectively prevent, ameliorate or treat immune-related disease (Jisun Lim et al., Glutathione dynamics determine the therapeutic efficacy of mesenchymal stem cells for graft-versus-host disease via CREB1-NRF2 pathway. Sci Adv. 2020 Apr 15;6(16):eaba1334.) (Eui Man Jeong et al., Real-Time Monitoring of Glutathione in Living Cells Reveals that High Glutathione Levels Are Required to Maintain Stem Cell Function. Stem Cell Reports. 2018 Feb 13;10(2):600-614.).

The definition of the control overlaps with that described above, and detailed description thereof will be omitted to avoid excessive complexity of the specification.

In the present invention, the "immune-related disease" is a disease caused by excessive activation and expression of various immune cells and inflammatory cells, and may be, for example, at least one selected from the group consisting of autoimmune disease, graft-versus-host disease, organ transplant rejection, asthma, atopy, idiopathic pulmonary fibrosis, and acute or chronic inflammatory diseases, without being limited thereto.

In addition, in the present invention, the "autoimmune disease" may be at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, polymyositis, ankylosing spondylitis, fibromyalgia, and polyarteritis nodosa, without being limited thereto.

Meanwhile, in the present invention, "prevention" may include without limitation any action that blocks, suppresses or delays the symptoms of a disease by using the pharmaceutical composition of the present invention.

In addition, in the present invention, "treatment" may include without limitation any action that alleviates or beneficially changes the symptoms of a disease by using the pharmaceutical composition of the present invention.

In the present invention, the pharmaceutical composition may be in the form of capsules, tablets, granules, injections, ointments, powders, or beverages, and the pharmaceutical composition may be for administration to humans.

For use, the pharmaceutical composition of the present invention may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be used for injection; and a base, an excipient, a lubricant, a preserving agent, and the like may be used for topical administration. In addition, the pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, or the like.

The routes of administration of the pharmaceutical composition according to the present invention include, but are not limited to, oral, intravenous, intramuscular, intra-arterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The pharmaceutical composition of the present invention may vary depending on various factors, including the activity of a specific compound used, the patient's age, body weight, general health, sex, diet, the time of administration, the route of administration, excretion rate, the drug content, and the severity of a specific disease to be prevented or treated. The dose of the pharmaceutical composition may be suitably selected by a person skilled in the art depending on the patient's condition and body weight, the severity of the disease, the form of drug, and the route and period of administration, and may be 0.0001 to 50 mg/kg/day or 0.001 to 50 mg/kg/day. The pharmaceutical composition may be administered once or several times a day. The dose is not intended to limit the scope of the present invention in any way. The pharmaceutical composition according to the present invention may be formulated as pills, sugar-coated tablets, capsules, liquids, gels, syrups, slurries, or suspensions.

In the present invention, details regarding the stem cells overlap with those described above, and thus detailed description thereof will be omitted to avoid excessive complexity of the specification.

### Advantageous Effects

The present invention provides a method capable of selecting high-quality stem cells which are suitable for use as a cell therapy product and have an increased expression level of, for example, VEGF or GSH gene, and a decreased expression level of, for example, CD71, IL-6, IL-8, or HMOX1 gene.

### Brief Description of Drawings

FIG. 1 shows that CD71 responds to oxidative stress applied to mesenchymal stem cells in Experimental Example 1.
FIG. 2 shows the results of comparing cell size and cell granularity between mesenchymal stem cells with low expression of CD71 and mesenchymal stem cells with high expression of CD71 in Experimental Example 2.
FIG. 3 shows the results of comparing the expression levels of VEGF, IL-6, IL-8, and HMOX1 genes between mesenchymal stem cells with low expression of CD71 and mesenchymal stem cells with high expression of CD71 in Experimental Example 3.
FIG. 4 shows the results of comparing the colony forming ability (the number of colony forming units) between mesenchymal stem cells with low expression of CD71 and mesenchymal stem cells with high expression of CD71 in Experimental Example 4.
FIG. 5 shows the results of confirming that cell activity is different between young-passage stem cells (P4), middle-passage stem cells (P8), and old-passage stem cells (P14) depending on the degree of senescence in Experimental Example 5.
FIG. 6 shows the results of comparing the expression level of CD71 gene between young-passage stem cells (P4) and old-passage stem cells (P14) in Experimental Example 5.
FIG. 7 shows the results of evaluating the idiopathic pulmonary fibrosis therapeutic effect of stem cells (CD71^{LOW}MSC) with low expression of CD71 and high expression of GSH in Experimental Example 6.
FIG. 8 shows the results of evaluating the anti-fibrotic and anti-inflammatory effects of stem cells (CD71^{LOW}MSC) with low expression of CD71 and high expression of GSH in Experimental Example 6.

### Best Mode

As a result of performing FACS analysis after applying oxidative stress to cells, it could be seen that the cells were sorted into two cell populations based on the GSH level (FR) and the CD71 expression level (Comp R1-A), and when the CD71 expression level was low, the GSH level was high, and thus MSCs with a low expression level of CD71 could be determined to be a high-quality MSC therapy product.

### Mode for Invention

Hereinafter, the present invention will be described in detail with reference to the following examples. However, the following examples are only for illustrating the present invention, and the scope of the present invention is not limited by the following examples.

### Examples

### [Experimental Example 11 FACS analysis for assessment of mesenchymal stem cell quality

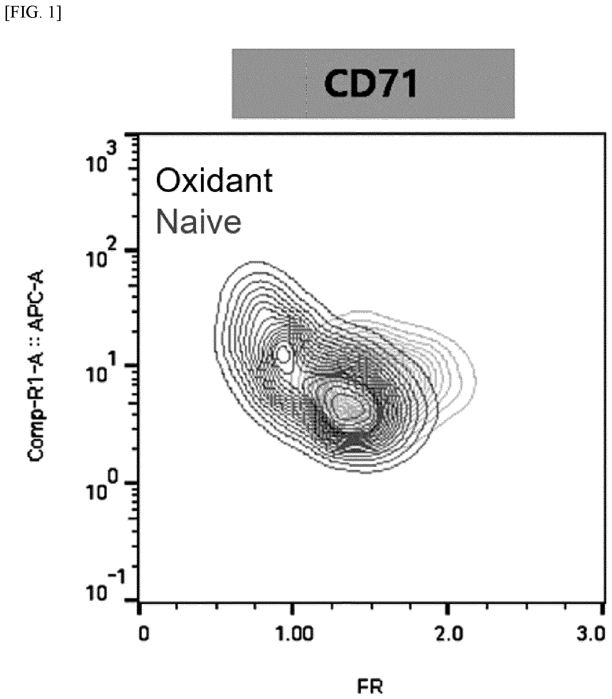

In order to assess the quality of mesenchymal stem cells (MSCs), marker candidates that respond to oxidative stress applied to the cells were identified. Specifically, MSCs were treated with a reagent capable of inducing oxidative stress and were stained with mitoFreSHtracer, which can measure the mitochondrial GSH level. Thereafter, the stained cells were detached, and then stained with the CD71 marker among the marker candidates and subjected to FACS analysis (BD Lyoplate^{™}, cat. #560747) to determine the GSH level and the expression level of each marker. The results are shown in FIG. 1.

As shown in FIG. 1, as a result of performing FACS analysis flow cytometry after applying oxidative stress to the cells, it could be seen that the cells were sorted into two cell populations according to the GSH level (FR) and the CD71 expression level (Comp R1-A), and when the CD71 expression level was low, the GSH level was high, and thus MSCs with a high expression level of CD71 could be determined to be a high-quality MSC therapy product.

### [Experimental Example 2] Analysis of cell size and cell granularity in cells with low expression level of CD71

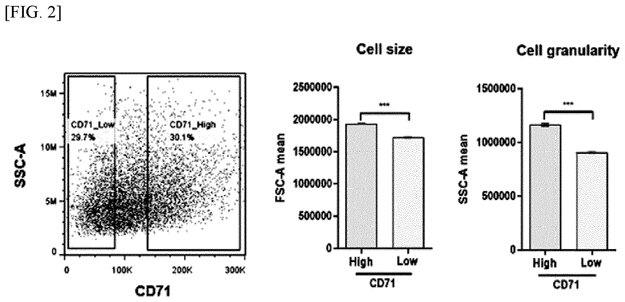

Those skilled in the art to which the present invention pertains generally recognize that stem cells having a smaller size and fewer intracellular granules (lower cell granularity) are "higher-quality stem cells". The "high quality" means that the stem cells are highly efficient and highly effective as a cell therapy product. Therefore, the present inventors examined whether there was a correlation between the cell size and the cell granularity of MSCs with a low expression level of CD71. To this end, the expression pattern of CD71 in MSCs was analyzed, and among the cells subjected to FACS analysis, the top 30% mesenchymal stem cells with high expression of CD71 (CD71_high) and the bottom 30% mesenchymal stem cells with low expression of CD71 (CD71_low) were selected, and cell size and cell granularity were compared between the two cell populations. The results are shown in FIG. 2.

As shown in FIG. 2, it could be confirmed that, among the cells subjected to FACS analysis, the top 30% mesenchymal stem cells with high expression of CD71 (CD71_high) had a smaller mean cell size and a lower cell granularity than the bottom 30% mesenchymal stem cells with low expression of CD71 (CD71_low).

Thereby, it could be confirmed that the mesenchymal stem cells with low expression of CD71 had a small cell size and a low cell granularity, and thus were determined to be of high quality.

### [Experimental Example 3] Analysis of changes in expression levels of major marker proteins in cells with low expression of CD71

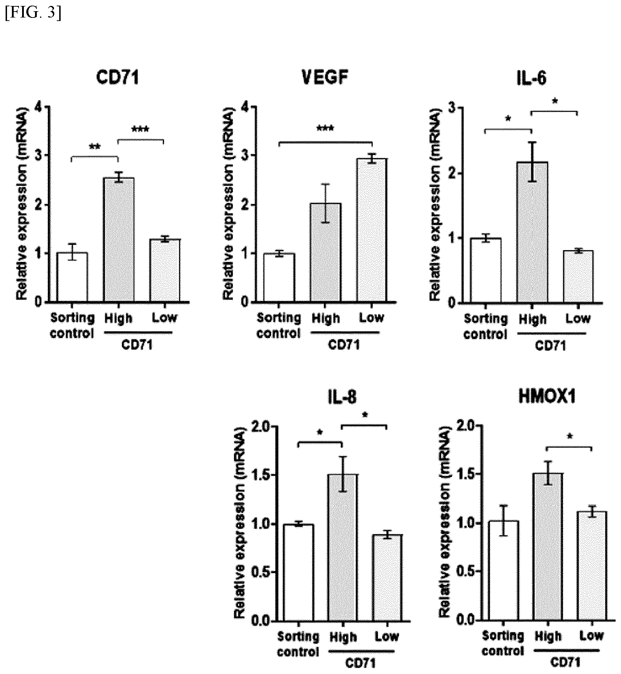

In order to confirm the quality of mesenchymal stem cells with low expression of CD71, the FACS analysis results in Experimental Example 1 were quantitatively analyzed. Specifically, among the cells subjected to FACS analysis, the top 30% mesenchymal stem cells with high expression of CD71 (CD71_high) and the bottom 30% mesenchymal stem cells with low expression of CD71 (CD71_low) were selected, and the expression levels of VEGF, IL-6, IL-8 and HMOX1 were compared between the two cell populations. The results are shown in FIG. 3.

As shown in FIG. 3, it could be confirmed that, among the cells subjected to FACS analysis, the top 30% mesenchymal stem cells with high expression of CD71 (CD71_high) had a significantly higher expression level of VEGF and significantly lower expression levels of IL-6, IL-8 and HMOX1 than the bottom 30% mesenchymal stem cells with low expression of CD71 (CD71_low).

It is generally known to those skilled in the art to which the present invention pertains that the expression of VEGF increases in young and healthy cells, and the expression of IL-6, IL-8 and HMOX1 increases as cells become senescent.

Therefore, it could be confirmed that, in the mesenchymal stem cells with low expression of CD71, the expression of VEGF increased and the expression of IL-6, IL-8 and HMOX1 expression decreased. Accordingly, these stem cells were determined to be of high quality.

### [Experimental Example 4] Analysis of colony forming ability of cells with low expression level of CD71

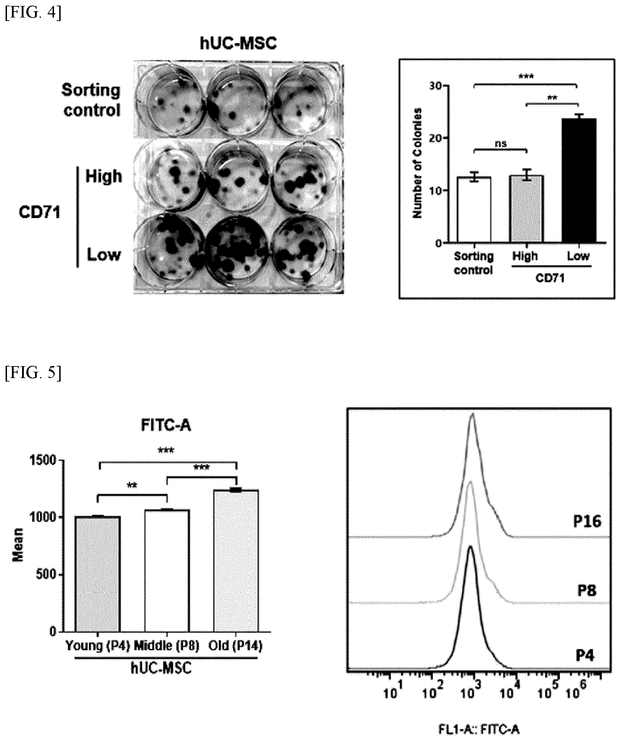

It is known that young and healthy stem cells in which the inherent self-renewal ability of the stem cells is well preserved have excellent colony forming ability (the number of colony forming units). Therefore, in order to assess the quality of mesenchymal stem cells with low expression of CD71, the colony forming ability was compared between the mesenchymal stem cells with low expression of CD71 (CD71_low) and the mesenchymal stem cells with high expression of CD71 (CD71_high), and the results are shown in FIG. 4.

As shown in FIG. 4, it could be seen that, among the cells subjected to FACS analysis, the colony forming ability was about two-fold higher in the bottom 30% mesenchymal stem cells with low expression of CD71 (CD71_low) than in the top 30% mesenchymal stem cells with high expression of CD71 (CD71_high). Accordingly, the bottom 30% mesenchymal stem cells with low expression of CD71 (CD71_low) could be determined to be of high quality.

### [Experimental Example 5] Analysis of CD71 expression level depending on degree of cell senescence

As stem cells become senescent, the sternness and therapeutic efficacy of the cells decrease. Thus, according to on the number of MSC passages, stem cells were divided into young-passage stem cells (P4), middle-passage stem cells (P8), and old-passage stem cells (P14). For the divided cells, lipofuscin (FITC_autofluorecence), which is known to accumulate during cell senescence, was measured, and it was confirmed that the cell activity was different depending on the degree of senescence. The results are shown in FIG. 5.

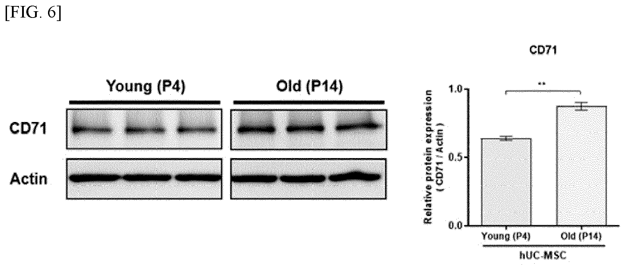

The expression levels of the CD71 marker protein in the young-passage stem cells (P4) and the old-passage stem cells (P14) were analyzed, and the results are shown in FIG. 6.

As shown in Figure 6, the expression of CD71 was significantly higher in the old-passage stem cells (P14) than in the young-passage stem cells (P4). Accordingly, it was confirmed that the CD71 marker can be used as a marker for selecting young, healthy, high-quality stem cells.

### [Experimental Example 6] Analysis of CD71 expression level

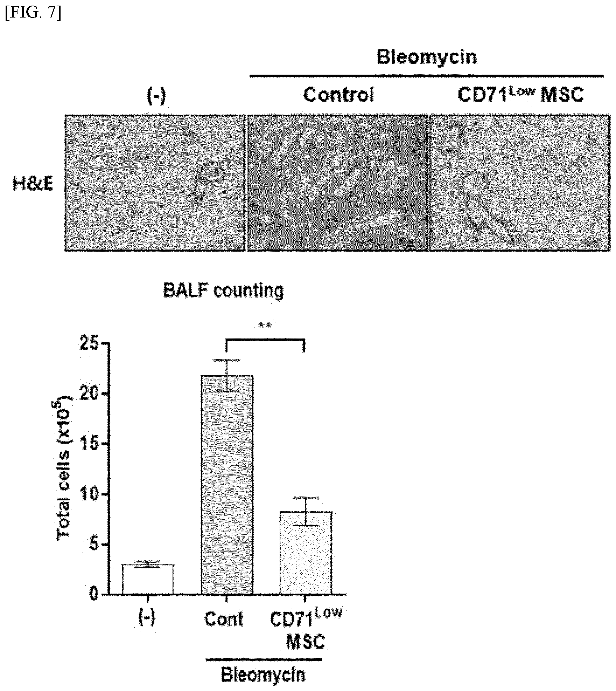

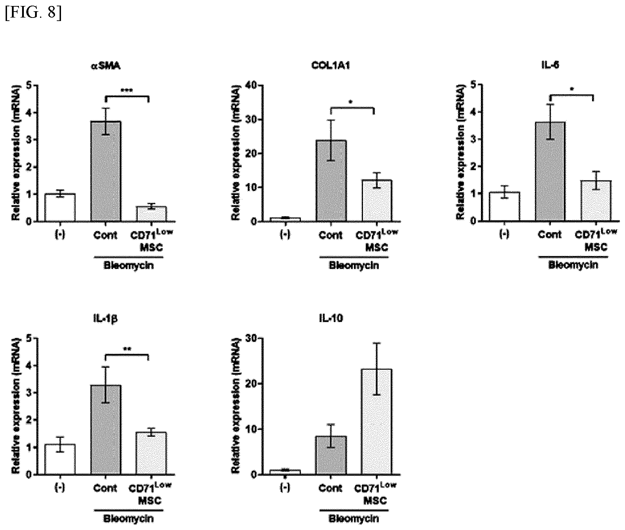

In Experimental Example 1, it was confirmed that the GSH level was high when the CD71 expression level was low. Thus, the idiopathic pulmonary fibrosis therapeutic effect of stem cells (CD71^{LOW}MSC) with low expression of CD71 and high expression of GSH was evaluated, and the results are shown in FIGS. 7 and 8.

As shown in FIG. 7, it could be seen that the infiltration of inflammatory cells significantly decreased in the tissue co-treated with bleomycin and CD71^{LOW}MSC compared to in the lesion tissue induced by bleomycin treatment.

In addition, as shown in FIG. 8, as a result of analyzing the mRNA level in lung tissue, it could be confirmed that the expression levels of the fibrosis markers aSMA and col1a1 and the proinflammatory cytokine IL-6 and IL-8 significantly decreased and the expression level of the anti-inflammatory cytokine IL-10 increased, in the lung tissue co-treated with bleomycin and CD71^{LOW}MSC compared to in the lesion tissue induced by bleomycin treatment. Accordingly, it could be confirmed that the CD71^{LOW}MSC cells with a low expression level of CD71 exhibited anti-fibrotic and anti-inflammatory effects.

As described above, as a result of assessing the quality of stem cells according to the present invention, mesenchymal stem cells with low expression of CD71 could be determined to be of higher quality than mesenchymal stem cells with high expression of CD71. Accordingly, it can be confirmed that, when mesenchymal stem cells with low expression of CD71 are selected and used for treatment, these stem cells will exhibit a further enhanced therapeutic effect.

### Industrial Applicability

In cell therapy technology using mesenchymal stem cells, it is important to use only high-purity and high-quality cells. This therapy technology has been attempted to treat intractable diseases such as various inflammatory diseases. Therefore, there is a need for markers for assessing the performance of mesenchymal stem cells for application to a mesenchymal stem cell therapy product.

The cell surface marker protein for selection of high-quality stem cells according to the present invention and the method of selecting high-quality stem cells using the marker protein make it possible to effectively select only high-quality cells, and thus the stem cells selected according to the present invention are expected to be actively used for the prevention or treatment of immune-related diseases such as autoimmune diseases, graft-versus-host diseases, organ transplant rejection, asthma, atopy, idiopathic pulmonary fibrosis, acute inflammation, and chronic inflammation.

### Sequence Listing Free Text

SEQ ID NO. 1: CD71 (Cluster of Differentiation 71) protein.

MMDQARSAFS NLFGGEPLSY TRFSLARQVD GDNSHVEMKL
AVDEEENADN NTKANVTKPK RCSGSICYGT IAVIVFFLIG FMIGYLGYCK
GVEPKTECER LAGTESPVRE EPGEDFPAAR RLYWDDLKRK LSEKLDSTDF
TGTIKLLNEN SYVPREAGSQ KDENLALYVE NQFREFKLSK VWRDQHFVKI

QVKDSAQNSV IIVDKNGRLV YLVENPGGYV AYSKAATVTG KLVHANFGTK
KDFEDLYTPV NGSIVIVRAG KITFAEKVAN AESLNAIGVL IYMDQTKFPI
VNAELSFFGH AHLGTGDPYT PGFPSFNHTQ FPPSRSSGLP NIPVQTISRA
AAEKLFGNME GDCPSDWKTD STCRMVTSES KNVKLTVSNV LKEIKILNIF
GVIKGFVEPD HYVVVGAQRD AWGPGAAKSG VGTALLLKLA QMFSDMVLKD
GFQPSRSIIF ASWSAGDFGS VGATEWLEGY LSSLHLKAFT YINLDKAVLG
TSNFKVSASP LLYTLIEKTM QNVKHPVTGQ FLYQDSNWAS KVEKLTLDNA
AFPFLAYSGI PAVSFCFCED TDYPYLGTTM DTYKELIERI PELNKVARAA
AEVAGQFVIK LTHDVELNLD YERYNSQLLS FVRDLNQYRA DIKEMGLSLQ
WLYSARGDFF RATSRLTTDF GNAEKTDRFV MKKLNDRVMR VEYHFLSPYV
SPKESPFRHV FWGSGSHTLP ALLENLKLRK QNNGAFNETL FRNQLALATW
TIQGAANALS GDVWDIDNEF

## Claims

1. A composition for assessing quality of stem cells, the composition containing an agent for measuring an expression level of a CD71 (Cluster of Differentiation 71) protein or a gene encoding the protein.

2. The composition according to claim 1, wherein, when the expression level of the CD71 protein or the gene encoding the protein is lower than a control, the stem cells are determined to be of high quality.

3. The composition according to claim 1, wherein the stem cells are mesenchymal stem cells (MSCs).

4. A kit for assessing quality of stem cells, the kit comprising the composition for assessing quality of stem cells according to any one of claims 1 to 3.

5. A method for selection of activated stem cells, the method comprising steps of:
obtaining stem cells; and
measuring an expression level of a CD71 (Cluster of Differentiation 71) protein or a gene encoding the protein in the stem cells.

6. The method according to claim 5, wherein, when the expression level of the CD71 protein or the gene encoding the protein in the stem cells is lower than a control, the stem cells are determined to be of high quality.

7. The method according to claim 6, wherein a phenotype of the activated stem cells determined to be of high quality is CD71^{low} stem cells.

8. The method according to claim 5, further comprising a step of measuring an oxidative stress of the stem cells.

9. The method according to claim 8, wherein the step of measuring the oxidative stress is performed by measuring a glutathione (GSH) level in the cells.

10. The method according to claim 5, wherein the stem cells are mesenchymal stem cells (MSCs).

11. The method according to claim 5, wherein an expression level of vascular endothelial growth factor (VEGF) in the selected stem cells is higher than a control.

12. The method according to claim 5, wherein an expression level of at least one of interleukin 6 (IL-6), interleukin 8 (IL-8), and heme oxygenase 1 (HMOX1) in the selected stem cells is lower than a control.

13. A cell therapy product containing, as an active ingredient, stem cells showing a CD71^{low} phenotype.

14. A pharmaceutical composition for preventing or treating immune-related disease, the pharmaceutical composition containing, as an active ingredient, stem cells showing a CD71^{low} phenotype.

15. The pharmaceutical composition according to claim 14, wherein the immune-related disease is at least one selected from the group consisting of autoimmune disease, graft-versus-host disease, organ transplant rejection, asthma, atopy, idiopathic pulmonary fibrosis, acute inflammation, and chronic inflammation.
